# EUROPEAN PATENT APPLICATION

(11) **EP 4 813 409 A1**
(43) Date of publication of application: **30.09.2026**
(21) Application number: 24894707.9
(22) Date of filing: 22.11.2024
(51) Int. Cl.: A61K 47/54, A61K 31/4745, A61K 31/52, A61K 31/4985, A61K 31/505, A61K 31/55, A61K 9/00, A61K 45/06, A61P 35/00

(54) **COMBINATION THERAPY OF ANTICANCER TREATMENT WITH TOLL-LIKE RECEPTOR 7 OR 8 AGONIST HAVING ACTIVE SITE TEMPORARILY INACTIVATED FOR CANCER TREATMENT**

(30) Priority: 23.11.2023 KR 20230164008
(71) Applicant: Progeneer Inc., Seoul 08380 (KR)
(72) Inventor: KANG, Taegyun, Gunpo-si, Gyeonggi-do 15826 (KR); KIM, Jeonghun, Seoul 08393 (KR); KIM, Sohyun, Suwon-si, Gyeonggi-do 16508 (KR); CHOI, Kyung Min, Incheon 21997 (KR); LEE, Inho, Uiwang-si, Gyeonggi-do 16042 (KR); KIM, Sehui, Suwon-si, Gyeonggi-do 16421 (KR); PARK, Yeji, Suwon-si, Gyeonggi-do 16417 (KR)
(74) Representative: Dehns
(86) International application number: PCT/KR2024/018697
(87) International publication number: WO 2025/110816

(57) **Abstract**

The present invention relates to a method for treating cancer, in which the anticancer effect is significantly enhanced by using a Toll-like receptor 7 or 8 agonist having the active site temporarily inactivated.

## Description

### [Technical Field]

This application claims priority to and the benefit of Korean Patent Application No. 10-2023-0164008, filed on November 23, 2023, the disclosures of the specification and drawings of which are incorporated herein by reference in their entirety.

The present invention relates to a combination therapy of anti-cancer treatment with a Toll-like receptor 7 or 8 agonist having an active site temporarily inactivated for treatment of cancer, and more particularly, to a kit for preventing or treating cancer, comprising: a) a first pharmaceutical composition comprising, as an active ingredient, a substance for anti-cancer treatment; and b) a second pharmaceutical composition comprising, as an active ingredient, a temporarily inactivated Toll-like receptor 7 or 8 agonist in which cholesterol is bonded to an active site via a cleavable linker, and to a method for treating cancer using the same.

### [Background Art]

Cancer is one of the diseases accounting for the largest proportion of deaths among modern people. Cancer is a disease caused by the transformation of normal cells due to gene mutations arising from various causes, and refers to malignant tumors that do not follow normal patterns of cell differentiation, proliferation, and growth. Cancer is characterized by "uncontrolled cell growth," and such abnormal cell growth forms a mass of cells called a tumor, which infiltrates surrounding tissues and, in severe cases, metastasizes to other organs of the body. Cancer is a refractory chronic disease that in many cases cannot be fundamentally cured even when treated with surgery, radiotherapy, and drug therapy, causing suffering to patients and ultimately leading to death. In particular, the global cancer incidence rate has been increasing by more than 5% every year due to the recent increase in the elderly population and environmental degradation. According to a World Health Organization (WHO) report, the number of people developing cancer is estimated to increase to 30 million within the next 25 years, of which 20 million are expected to die from cancer. Although various treatment methods are continuously being developed to effectively treat cancer, the development of effective treatment methods capable of treating various types of cancer is still insufficient.

Meanwhile, an immune response is a series of reactions caused by activated immune cells against exogenous and endogenous substances, namely antigens. When microorganisms including bacteria and viruses or foreign substances enter the living body, immune cells recognize them and become activated, and secrete factors such as cytokines to induce an inflammatory response. Recently, research on mechanisms in the innate immune response stage, which act non-specifically at an early stage of infection, has been actively conducted. Among these, Toll-like receptors (TLR) are genes capable of recognizing pathogens at an early stage of inflammation and are known to induce immune responses by recognizing plasma membrane components, nucleic acid components, and the like of pathogens. Utilizing this, studies on various TLR ligands for activating immune cells are being actively conducted (US Patent Publication 2012-0294885). Among these, substances based on TLR 7/8 agonists are being used as immune adjuvants to induce cellular immune responses, and imiquimod, resiquimod, dactolisib, gardiquimod, sumanirole, motolimod, vesatolimod, loxoribine, SM360320, CL264, 3M-003, imidazoquinoline (IMDQ), Compound 54, and the like are known. These TLR 7/8 agonists act on TLR 7/8 within the endosome and are known to effectively induce not only humoral immunity but also cellular immunity. However, these TLR 7/8 agonists are difficult to be dispersed in aqueous solutions due to their molecular structures. Furthermore, since TLR 7/8 agonists are soluble only in particular organic solvents such as dimethyl sulfoxide (DMSO) and methanol and are not soluble in commonly used organic solvents, there are limitations in preparing immune-activating substances in various formulations. Therefore, TLR 7/8 agonists are commercialized in the form of cream formulations (e.g., Aldara^{®} cream) mixed with various surfactants. In some studies, to overcome these problems, TLR 7/8 agonists were prepared in the form of salts to be soluble in aqueous solutions. However, TLR 7/8 agonists prepared in salt forms are absorbed into blood vessels in the body and induce a systemic immune response, causing many side effects (e.g., cytokine storm and various non-specific hypersensitive immune responses), and thus using the salt forms is difficult. To address these side effect issues, a temporarily inactivated TLR 7/8 agonist was developed by binding cholesterol to the active site of a TLR 7/8 agonist with a cleavable linker (Korean Patent Publication 10-2323540).

Therefore, when a TLR 7/8 agonist which is not absorbed into blood vessels in the body, suppresses non-specific immune responses, and can be prepared in various formulations is applied to the treatment of cancer, an effective cancer treatment method that can be applied to various types of cancer through immune-activating action while reducing side effects is expected to be developed.

### [Disclosure]

### [Technical Problem]

An object of the present invention is to provide, in order to solve the problems of the conventional art as described above, a pharmaceutical composition for preventing or treating cancer, comprising, as an active ingredient, a temporarily inactivated Toll-like receptor 7 or 8 agonist in which cholesterol is bonded to an active site via a cleavable linker, wherein the pharmaceutical composition is administered 1 to 20 days after anti-cancer treatment, a kit for preventing or treating cancer, comprising: a) a first pharmaceutical composition comprising, as an active ingredient, a substance for anti-cancer treatment; and b) a second pharmaceutical composition comprising, as an active ingredient, a temporarily inactivated Toll-like receptor 7 or 8 agonist in which cholesterol is bonded to an active site via a cleavable linker, a use thereof, and a method for treating cancer using the same.

However, technical problems to be solved in the present invention are not limited to the above-described problems, and other problems which are not described herein will be fully understood by those of ordinary skill in the art from the following descriptions.

### [Technical Solution]

The present invention provides a pharmaceutical composition for combination administration for preventing or treating cancer, comprising, as an active ingredient, a temporarily inactivated Toll-like receptor 7/8 agonist in which cholesterol is bonded to an active site via a cleavable linker, wherein the pharmaceutical composition is administered 1 to 20 days after anti-cancer treatment.

Additionally, the present invention provides a kit for preventing or treating cancer, comprising: a) a first pharmaceutical composition comprising, as an active ingredient, a substance for anti-cancer treatment; and b) a second pharmaceutical composition comprising, as an active ingredient, a temporarily inactivated Toll-like receptor 7/8 agonist in which cholesterol is bonded to an active site via a cleavable linker, wherein the first pharmaceutical composition and the second pharmaceutical composition are sequentially administered. Preferably, in the kit, the second pharmaceutical composition is administered 1 to 20 days after the first pharmaceutical composition is administered, such that tumor lysates generated by the first pharmaceutical composition act as antigens and may significantly increase the immuno-oncological effect of the second pharmaceutical composition. The kit may be for single administration or for multiple administrations, and the numbers of administrations of the first pharmaceutical composition and the second pharmaceutical composition may be identical or different. Preferably, the kit may be for 1 to 15 administrations, 1 to 13 administrations, 1 to 11 administrations, 1 to 9 administrations, 1 to 7 administrations, 1 to 5 administrations, 1 to 3 administrations, or 1 to 2 administrations. Additionally, preferably, the first pharmaceutical composition may be for 1 to 15 administrations, 1 to 13 administrations, 1 to 11 administrations, 1 to 9 administrations, 1 to 7 administrations, 1 to 5 administrations, 1 to 3 administrations, or 1 to 2 administrations, and preferably, the second pharmaceutical composition may be administered the same number of times as the first pharmaceutical composition, may be administered 1 to 10 times per single administration of the first pharmaceutical composition, may be administered 1 to 9 times, may be administered 1 to 8 times, may be administered 1 to 7 times, may be administered 1 to 6 times, may be administered 1 to 5 times, may be administered 1 to 4 times, may be administered 1 to 3 times, or may be administered 1 to 2 times.

In one specific embodiment of the present invention, the anti-cancer treatment may preferably be anti-cancer drug treatment, radiotherapy, oncolytic virus treatment, or the like, and the substance for anti-cancer treatment may be an anti-cancer drug, an oncolytic virus, a radioisotope, or the like, but is not limited thereto as long as it is an anti-cancer treatment method or a substance for anti-cancer treatment that may be used for treatment of cancer. Preferably, there is no limitation as long as it is an anti-cancer treatment method or a substance for anti-cancer treatment capable of generating tumor lysates by killing cancer cells and inducing destruction of tumor tissue.

In another specific embodiment of the present invention, the anti-cancer drug may preferably be doxorubicin, cisplatin, carboplatin, nedaplatin, goserelin, medroxyprogesterone, cyproterone, vinorelbine, cabazitaxel, denosumab, gemcitabine, capecitabine, oxaliplatin, vorinostat, entinostat, 5-fluorouracil, taxol, topotecan, irinotecan, or pharmaceutically acceptable salts thereof, each of which induces cancer cell death, but is not limited thereto as long as it is any approved drug intended for treatment of cancer (National Cancer Institute, https://www.cancer.gov/about-cancer/treatment/drugs).

In yet another specific embodiment of the present invention, the oncolytic virus may preferably be one or more selected from the group consisting of herpesvirus, adenovirus, vaccinia virus, poliovirus, measles virus, vesicular stomatitis virus, reovirus, and genetically modified viruses thereof, but is not limited thereto as long as it is a virus capable of lysing tumors to generate tumor lysates.

In yet another specific embodiment of the present invention, the radiotherapy may be treatment of cancer using high-energy radiation such as X-rays, gamma rays, electron beams, and proton beams, or may be treatment of cancer using a radioisotope. The radioisotope is not limited as long as it is a radioisotope used for treatment of cancer, but may preferably be iridium-192.

In yet another specific embodiment of the present invention, the administration may be performed by a method such as intrathecal injection, intravenous injection, intra-articular injection, subcutaneous injection, intradermal injection, intramuscular injection, intratumoral injection, intraocular injection, or intraperitoneal injection, and preferably, the first pharmaceutical composition may be administered in the form of intravenous injection, and the second pharmaceutical composition may be administered in the form of intravenous injection, intradermal injection, intratumoral injection, or subcutaneous injection. However, the present invention is not limited thereto as long as it is a form generally used for administration of an anti-cancer drug or a Toll-like receptor 7 or 8 agonist.

In yet another specific embodiment of the present invention, the cancer may be breast cancer, colorectal cancer, rectal cancer, lung cancer, colon cancer, thyroid cancer, oral cancer, pharyngeal cancer, laryngeal cancer, cervical cancer, brain cancer, ovarian cancer, bladder cancer, kidney cancer, liver cancer, pancreatic cancer, prostate cancer, skin cancer, tongue cancer, uterine cancer, gastric cancer, bone cancer, blood cancer, head and neck cancer, bone cancer, vaginal cancer, esophageal cancer, lymphoma, gallbladder cancer, endocrine gland cancer, adrenal cancer, melanoma, or the like, but is not limited thereto as long as it is a type of cancer that may be treated using an anti-cancer drug and a Toll-like receptor 7 or 8 agonist. More specifically, since the composition or kit of the present invention generates tumor lysates by using various anti-cancer treatments that induce death of cancer cells and allows the tumor lysates to act as antigens, the composition or kit of the present invention may be applied to treatment of various cancers regardless of the type of cancer, and since the Toll-like receptor 7 or 8 agonist acts as an adjuvant to significantly increase immuno-oncological activity, the composition or kit of the present invention may be used not only for treatment of early-stage cancer but also for treatment of advanced cancer, and furthermore, may also be used to effectively suppress recurrence of cancer by continuously inducing an immune response in the body like a vaccine.

In yet another specific embodiment of the present invention, the Toll-like receptor 7 or 8 agonist may preferably be at least one selected from the group consisting of an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist, and more preferably, may be imiquimod, resiquimod, dactolisib, gardiquimod, sumanirole, motolimod, vesatolimod, loxoribine, SM360320, CL264, 3M-003, IMDQ, Compound 54, or the like, but is not limited thereto as long as it is a Toll-like receptor 7 or 8 agonist in which cholesterol is chemically bonded to an active site, that is, NH2, to exhibit a temporarily inactivated form.

In yet another specific embodiment of the present invention, the cleavable linker may preferably comprise at least one bond selected from the group consisting of carbamate, disulfide, ester, peptide, azide, or a combination thereof, but is not limited thereto as long as the chemical bond with cholesterol is cleaved in response to the tumor microenvironment or the enzymes and pH of intracellular endosomes and lysosomes, such that the active site of the Toll-like receptor 7 or 8 agonist is exposed and the function of the Toll-like receptor 7 or 8 agonist may be restored.

In yet another specific embodiment of the present invention, the pharmaceutical composition or kit may further comprise an immune checkpoint inhibitor, or a third pharmaceutical composition comprising, as an active ingredient, an immune checkpoint inhibitor. The immune checkpoint inhibitor may preferably be anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, anti-TIGIT, or the like, but is not limited thereto as long as it is an immune checkpoint inhibitor used as an immuno-oncological agent.

In yet another specific embodiment of the present invention, the pharmaceutical composition or kit is characterized by inhibiting proliferation, metastasis, recurrence, or the like of cancer, or inhibiting resistance to an anti-cancer treatment regimen, but is not limited thereto as long as it is part of a generally used cancer treatment method.

In yet another specific embodiment of the present invention, the pharmaceutical compositions may be prepared in the form of nanoparticles in order to facilitate administration and formulation, but this is merely for facilitating administration and formulation and is not related to therapeutic effects, and thus is not limited thereto. The nanoparticles may be nanoliposomes, nanomicelles, solid nanoparticles, nanoemulsions, or the like, but are not limited thereto.

Additionally, the present invention provides a method for preventing or treating cancer, comprising administering the pharmaceutical composition to a subject in need thereof in a therapeutically effective amount.

Additionally, the present invention provides a method for preventing or treating cancer, comprising administering the pharmaceutical composition to a subject in need thereof in a therapeutically effective amount using the kit.

Additionally, the present invention provides a use of the pharmaceutical composition or kit for preventing or treating cancer.

Additionally, the present invention provides a use of a temporarily inactivated Toll-like receptor 7 or 8 agonist, in which cholesterol is bonded to an active site via a cleavable linker, for producing a medicament used to increase an anti-cancer effect by administering the temporarily inactivated Toll-like receptor 7 or 8 agonist 1 to 20 days after anti-cancer treatment.

### [Advantageous Effects]

It was confirmed that, in the pharmaceutical composition and/or kit according to the present invention, after primary anti-cancer treatment is performed by first inducing destruction of tumor tissue through administration of a chemical anti-cancer agent, radiotherapy, administration of an oncolytic virus, or the like, tumor lysates generated thereby act as antigens, and at this time, by secondarily administering a Toll-like receptor 7 or 8 agonist having an active site temporarily inactivated, the effect of treatment of cancer may be significantly increased by significantly increasing an immune activation effect. Additionally, it was confirmed that the anti-cancer effect may be further significantly increased by intratumorally administering the Toll-like receptor 7 or 8 agonist having an active site temporarily inactivated, or by additionally administering an immune checkpoint inhibitor. Therefore, the pharmaceutical composition and/or kit according to the present invention is expected to be effectively applied to treatment effects of various cancers.

### [Description of Drawings]

FIG. 1 is a view showing results confirming the effects of monotherapy and combination therapy in a syngeneic mouse tumor model according to one example of the present invention based on tumor size.
FIG. 2 is a view showing results confirming the effects of monotherapy and combination therapy in a syngeneic mouse tumor model according to one example of the present invention based on the number of MDSC cells in the spleen.
FIG. 3 is a view showing results confirming the effects of monotherapy and combination therapy in a syngeneic mouse tumor model according to one example of the present invention based on the number of T cells in the spleen.
FIG. 4 is a view showing results confirming the effects of monotherapy and combination therapy in a syngeneic mouse tumor model according to one example of the present invention based on the numbers of T cells and NK cells in tumor tissue.
FIG. 5 is a view showing results confirming the combination administration effect of a Toll-like receptor 7 or 8 agonist having an active site temporarily inactivated, an anti-cancer drug, and an immune checkpoint inhibitor in a syngeneic mouse tumor model according to one example of the present invention based on tumor size.
FIG. 6 is a view showing results confirming the combination administration effect on various cancer types according to one example of the present invention.
FIG. 7 is a view showing results confirming the anti-cancer effect in primary retransplanted cancer according to one example of the present invention.
FIG. 8 is a view showing results confirming the anti-cancer effect in secondary retransplanted cancer according to one example of the present invention.
FIG. 9 is a view showing results confirming the anti-cancer effect according to the dose of the temporarily inactivated Toll-like receptor 7 or 8 agonist according to one example of the present invention.
FIG. 10 is a view showing results confirming the anti-cancer effect according to the administration method of the temporarily inactivated Toll-like receptor 7 or 8 agonist according to one example of the present invention.
FIG. 11 is a view showing results confirming the anti-cancer effect according to the administration dose and administration schedule of the temporarily inactivated Toll-like receptor 7 or 8 agonist according to one example of the present invention.
FIG. 12 is a view showing results confirming the anti-cancer effect according to the administration dose and administration schedule of the temporarily inactivated Toll-like receptor 7 or 8 agonist according to one example of the present invention.
FIG. 13 is a view showing experimental results of performing anti-cancer treatment at intervals of one week according to one example of the present invention.
FIG. 14 is a view showing results confirming infiltration of immune cells into tumors after anti-cancer treatment according to one example of the present invention. The white bar in the figure indicates 100 µm.
FIG. 15 is a view briefly showing the therapeutic mechanism of the kit of the present invention.

### [Best Modes]

Throughout the present specification, when a part is described as "including" a certain constituent element, this means that other constituent elements are not excluded but may be further included unless specifically stated otherwise.

As used herein, the terms of degree such as "about", "substantially", and the like are used to mean values at or close to the stated values when manufacturing and material tolerances inherent in the stated meaning are presented, and are used to prevent an unscrupulous infringer from unfairly exploiting the disclosed content in which exact or absolute values are stated in order to facilitate understanding of the present invention. Additionally, throughout the present specification, the phrase "step of" does not mean "step for".

Throughout the present specification, the term "combination thereof" included in a Markush-type expression means one or more mixtures or combinations selected from the group consisting of the constituent elements described in the Markush-type expression, and means including one or more selected from the group consisting of the constituent elements.

In the present specification, the "Toll-like receptor 7 or 8 agonist having an active site temporarily inactivated" is characterized in that cholesterol is bonded to the active site via a cleavable linker such that the immune activation function is temporarily inhibited. The inhibition may mean that the function of the active site of the Toll-like receptor 7 or 8 agonist is delayed. The cleavable linker is not limited in form as long as it may be naturally separated by the tumor microenvironment and/or the intracellular physiological environment, particularly the physiological environment of endosomes and lysosomes, such as low pH, enzymes, and glutathione, such that the function of the Toll-like receptor 7 or 8 agonist may be restored.

In the present specification, "cholesterol" collectively refers to steroid-based organic materials having hydrophobic properties as a type of lipid, and the cholesterol may comprise various analogs based on the cholesterol structure and compounds obtained by chemically modifying a part of cholesterol. Preferably, the cholesterol may comprise bile acids (cholic acid, deoxycholic acid, lithocholic acid, chenodeoxycholic acid), Vitamin D, steroid hormones (testosterone, estradiol, cortisol, aldosterone, prednisolone, prednisone), or the like, but is not limited thereto. Additionally, the cholesterol is a material that helps position the Toll-like receptor 7 or 8 agonist on the surface and inside of nanoparticles in various forms, and may be replaced with a lipid material having a similar function, for example, a natural lipid such as phospholipids or a synthetic lipid.

In the present specification, the term "Toll-like receptor 7/8 agonist-based materials" refers to agonists of Toll-like receptor 7 or 8, and may be selected from the group consisting of imidazoquinoline-based compounds, 8-hydroxyadenine-based compounds, pteridone-based compounds, 2-aminopyrimidine-based compounds, benzoazepine-based compounds, and 7-thia-8-oxoguanosine-based compounds, and the imidazoquinoline-based compounds comprise compounds of the type described in WO 2018 196823, WO 2011 049677, WO 2011 027022, WO 2017 102652, WO 2019 040491, and the like, or pharmaceutically acceptable salts thereof, but are not limited thereto. Additionally, the 8-hydroxyadenine-based compounds comprise compounds of the type described in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, WO 2018 041763, and the like, or pharmaceutically acceptable salts thereof, but are not limited thereto. The pteridone-based compounds comprise compounds of the type described in US 2010 0143301, WO 2016 007765, WO 2016 044182, WO 2017 035230, WO 2017 219931, WO 2011 057148, CN 1087 94486, and the like, or pharmaceutically acceptable salts thereof, but are not limited thereto. The 2-aminopyrimidine-based compounds comprise compounds of the type described in WO 2010 133885, WO 2012 066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319, WO 2013 117615, and the like, or pharmaceutically acceptable salts thereof, but are not limited thereto. The benzoazepine-based compounds comprise compounds of the type described in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112, WO 2017 197624, and the like, or pharmaceutically acceptable salts thereof, but are not limited thereto. The 7-thia-8-oxoguanosine-based compounds comprise compounds of the type described in WO 2016 180691, WO 2016 055553, WO 2016 180743, WO 2016 091698, and the like, or pharmaceutically acceptable salts thereof, but are not limited thereto. In addition, Toll-like receptor 7 or 8 compounds described in PCT/US2009/035563, PCT/US2015/028264, PCT/US2016/020499, WO 2015 023598, PCT/US2015/039776, and the like, or pharmaceutically acceptable salts thereof, may be comprised, but are not limited thereto, and all Toll-like receptor 7 or 8 agonists that may be readily conceived and used by those skilled in the art are comprised.

In the present specification, "co-administration" refers to administration of a conjugate of a Toll-like receptor 7 or 8 agonist and cholesterol together with various materials such as an antigen, an immune checkpoint inhibitor, an immune adjuvant, an immune activation material, and a chemical anti-cancer agent for treatment of cancer, and the type and form thereof are not limited. As used herein, the term "combination" may be achieved by simultaneously, sequentially, or separately administering (treating) the individual components of a therapeutic regimen. A combination therapeutic effect may be obtained by performing two or more anti-cancer therapies simultaneously, sequentially, or alternately at regular or irregular intervals, and the combination therapy is not limited thereto, but may be defined, for example, as providing a synergistic effect while being therapeutically superior to the efficacy obtainable by administering one or the remaining components of the combination therapy at a conventional dose, as measured by the degree of response, response rate, time to disease progression, or survival period.

In the present specification, the anti-cancer agent is not limited as long as it is a compound used for treatment of cancer known to those skilled in the art, and examples thereof comprise Paclitaxel, Docetaxel, 5-Fluorouracil, Alendronate, Doxorubicin, Simvastatin, Hydrazinocurcumin, Amphotericin B, Ciprofloxacin, Rifabutin, Rifampicin, Efavirenz, Cisplatin, Theophyline, Pseudomonas exotoxin A, Zoledronic acid, Trabectedin, Siltuximab, Dasatinib, Sunitinib, Apatinib, 5,6-Dimethylxanthenone-4-acetic acid, Silibinin, PF-04136309, Trabectedin, Carlumab, BLZ945, PLX3397, Emactuzumab, AMG-820, IMC-CS4, GW3580, PLX6134, N-acetyl-l-cystein, Vitamin C, bortezomib, aspirin, salicylates, Indolecarboxamide derivatives, quinazoline analogues, Thalidomide, prostaglandin metabolites, 2ME2, 17-AAG, Camptothecin, Topotecan, Pleurotin, 1-methylpropyl, 2-imidazolyl dissulphide, Tadalafil, Sildenafil, L-AME, Nitroaspirin, Celecoxib, NOHA, Bardoxolone methyl, D,L-1-methyl-tryptophan, Gemcitabine, Axitinib, Sorafenib, Cucurbitacin B, JSI-124, Anti IL-17 antibodies, Anti-glycan antibodies, Anti-VEGF antibodies, Bevacizumab, Antracycline, Tasquinimod, Imatinib, cyclophosphamide, and the like, but is not limited thereto.

In the present specification, "antigen" refers to a material having the ability to generate an immune response in a host. The antigen may be recognized by and bind to an antibody. The antigen may be derived from within the body or from an external environment, and may preferably be tumor lysates generated by anti-cancer treatment.

In the present specification, "prevention" means any act of suppressing cancer or delaying onset thereof by administration of the composition according to the present invention.

In the present specification, "treatment" means any act by which symptoms of cancer are improved or beneficially changed by administration of the composition according to the present invention.

In the present specification, "individual or subject" refers to a target to which the composition of the present invention may be administered, and is preferably a mammal including a human, but is not limited thereto. The "subject in need thereof" may preferably mean a subject having cancer or a subject at high risk of developing cancer and requiring treatment, but is not limited thereto.

In the present specification, "active ingredient" is used interchangeably with active drug, active component, active formulation, drug, and therapeutic agent, and refers to any material used to prevent, alleviate, improve, or treat a target disease.

In the present specification, "cancer" collectively refers to various blood cancers, malignant solid tumors, and the like, which may locally expand through invasion and systemically expand through metastasis. Although not particularly limited thereto, specific examples of cancer comprise colorectal cancer, adrenal cancer, bone cancer, brain cancer, breast cancer, bronchial cancer, colon cancer and/or rectal cancer, gallbladder cancer, gastrointestinal cancer, head and neck cancer, laryngeal cancer, liver cancer, lung cancer, neurogenic cancer, pancreatic cancer, prostate cancer, parathyroid cancer, skin cancer, gastric cancer, thyroid cancer, and the like. Other examples of cancer comprise adenocarcinoma, adenoma, basal cell carcinoma, cervical dysplasia and carcinoma in situ, Ewing sarcoma, squamous cell carcinoma, acinar cell carcinoma, malignant brain tumor, blastoma, intestinal ganglioneuroma, hyperplastic corneal neuroma, islet cell carcinoma, Kaposi cancer, leiomyoma, leukemia, lymphoma, malignant carcinoid, malignant melanoma, malignant hypercalcemia, Marpanoid habitus cancer, hydatidiform cancer, metastatic skin cancer, mucosal neuroma, myelodysplastic syndrome, myeloma, filamentous sarcoma, neuroblastoma, osteosarcoma, osteogenic and other sarcomas, ovarian cancer, chromaffin cell tumor, polycythemia vera, primary brain tumor, small cell lung cancer, ulcerative and papillary squamous epithelial carcinoma, seminoma, soft tissue sarcoma, retinoblastoma, rhabdomyoblastoma, renal cell tumor or renal cell carcinoma, reticular cell sarcoma, and Wilms tumor. Additionally, astrocytoma, gastrointestinal stromal tumor (GIST), glioma or glioblastoma, renal cell carcinoma (RCC), hepatocellular carcinoma (HCC), pancreatic neuroendocrine cancer, and the like are included.

In the present specification, "active component", "active drug", "active ingredient", "active formulation", "drug", and "therapeutic agent" are used interchangeably herein, and refer to any material used to prevent, alleviate, or treat a target disease.

In the present specification, the "pharmaceutical composition" may be characterized as being in the form of a capsule, tablet, granule, injection, ointment, powder, or beverage, and the pharmaceutical composition may be characterized as being intended for humans. Although the pharmaceutical composition is not limited thereto, it may be formulated and used in the form of an oral dosage form such as powder, granule, capsule, tablet, and aqueous suspension, an external preparation, a suppository, and a sterile injectable solution according to conventional methods. The pharmaceutical composition of the present invention may comprise a pharmaceutically acceptable carrier. As the pharmaceutically acceptable carrier, for oral administration, a binder, glidant, disintegrant, excipient, solubilizer, dispersant, stabilizer, suspending agent, colorant, flavoring agent, or the like may be used, in the case of an injection, a buffer, preservative, pain-relieving agent, solubilizer, isotonic agent, stabilizer, or the like may be mixed and used, and in the case of topical administration, a base, excipient, lubricant, preservative, or the like may be used. The formulation of the pharmaceutical composition of the present invention may be variously prepared by mixing with the pharmaceutically acceptable carriers as described above. For example, for oral administration, it may be prepared in the form of a tablet, troche, capsule, elixir, suspension, syrup, wafer, or the like, and in the case of an injection, it may be prepared in the form of a unit dosage ampule or a multiple dosage form. In addition, it may be formulated as a solution, suspension, tablet, capsule, sustained-release formulation, or the like.

Meanwhile, examples of carriers, excipients, and diluents suitable for formulation may comprise lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like. In addition, fillers, anti-caking agents, lubricants, wetting agents, flavoring agents, emulsifiers, preservatives, and the like may be further included.

The route of administration of the pharmaceutical composition according to the present invention is not limited thereto, but includes oral, intravenous, intramuscular, intra-arterial, intraosseous, intrathecal, intracardiac, transdermal, subcutaneous, intraperitoneal, intranasal, enteral, topical, sublingual, or rectal administration. Oral or parenteral administration is preferred. As used herein, the term "parenteral" includes subcutaneous, intradermal, intravenous, intramuscular, intra-articular, intrabursal, intrasternal, intrathecal, intralesional, and intracranial injection or infusion techniques. The pharmaceutical composition of the present invention may also be administered in the form of a suppository for rectal administration.

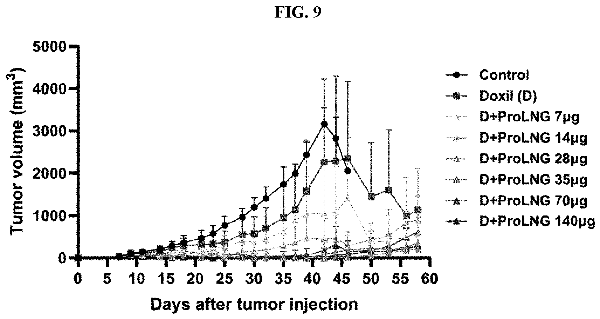

The pharmaceutical composition of the present invention may vary depending on various factors including the activity of the specific compound used, age, body weight, general health condition, sex, diet, administration time, route of administration, excretion rate, drug combination, and the severity of the specific disease to be prevented or treated, and the dosage of the pharmaceutical composition may vary depending on the condition of the patient, body weight, severity of the disease, drug form, route of administration, and duration of administration, but may be appropriately selected by those skilled in the art, and may be administered in an amount of 0.0001 to 500 mg/kg or 0.001 to 500 mg/kg per day. Preferably, as shown in FIG. 9, a pharmaceutically effective amount is administered, and the pharmaceutically effective amount refers to an amount exhibiting a response greater than that of a negative control group and means an amount sufficient to treat a disease at a reasonable benefit/risk ratio applicable to medical treatment, and the effective dosage level cannot be limited to a specific amount because it may be determined according to factors including the type and severity of the patient's disease, the activity of the drug, sensitivity to the drug, administration time, route of administration, excretion rate, treatment duration, concomitant medications, and other factors well known in the medical field. Preferably, it means an amount sufficient to treat or prevent the target disease. Administration may be performed once a day or may be divided into multiple administrations. The above dosage does not limit the scope of the present invention in any respect. The pharmaceutical composition according to the present invention may be formulated as a pill, sugar-coated tablet, capsule, liquid preparation, gel, syrup, slurry, or suspension.

In the present specification, the term "kit" refers to a device capable of separately administering an anticancer agent and a Toll-like receptor 7 or 8 agonist, and may comprise each pharmaceutical composition in the form of a separately packaged syringe, or may be in a form in which the two pharmaceutical compositions are packaged together. Alternatively, it may further comprise other immune checkpoint inhibitors, local anesthetics, and the like. However, there is no limitation as long as it is in a form in which the pharmaceutical composition comprising an anticancer agent and the pharmaceutical composition comprising a Toll-like receptor 7 or 8 agonist as an active ingredient can be separately administered by controlling the administration time, administration amount, administration method, and the like.

Hereinafter, preferred Examples are provided to facilitate understanding of the present invention. However, the following Examples are provided only to facilitate understanding of the present invention, and the scope of the present invention is not limited by the following Examples.

### [Examples]

### Example 1: Synthesis of conjugates of Toll-like receptor (TLR) 7/8 agonist and cholesterol

Various TLR 7/8 agonists (imidazoquinoline, 8-hydroxyadenine, pteridone, 2-aminopyrimidine, benzoazepine, 7-thia-8-oxoguanosine series, and the like) conjugated with cholesterol are prepared by chemical reactions as shown in Reaction Scheme 1 or 2. These may be prepared by allowing the amine group (NH₂), which is the active site of the TLR 7/8 agonists or derivatives thereof, to react with cholesterol or a cholesterol analog capable of forming a carbamate bond, a disulfide bond, an ester bond, a peptide bond, an azide bond, and the like. The analog collectively refers to similar compounds obtained by chemically modifying a portion of the TLR 7/8 agonist or cholesterol. More specifically, TLR 7/8 agonist conjugates with a temporarily inactivated active site were prepared using the same method disclosed in Korean Patent Publication No. 10-2323540.

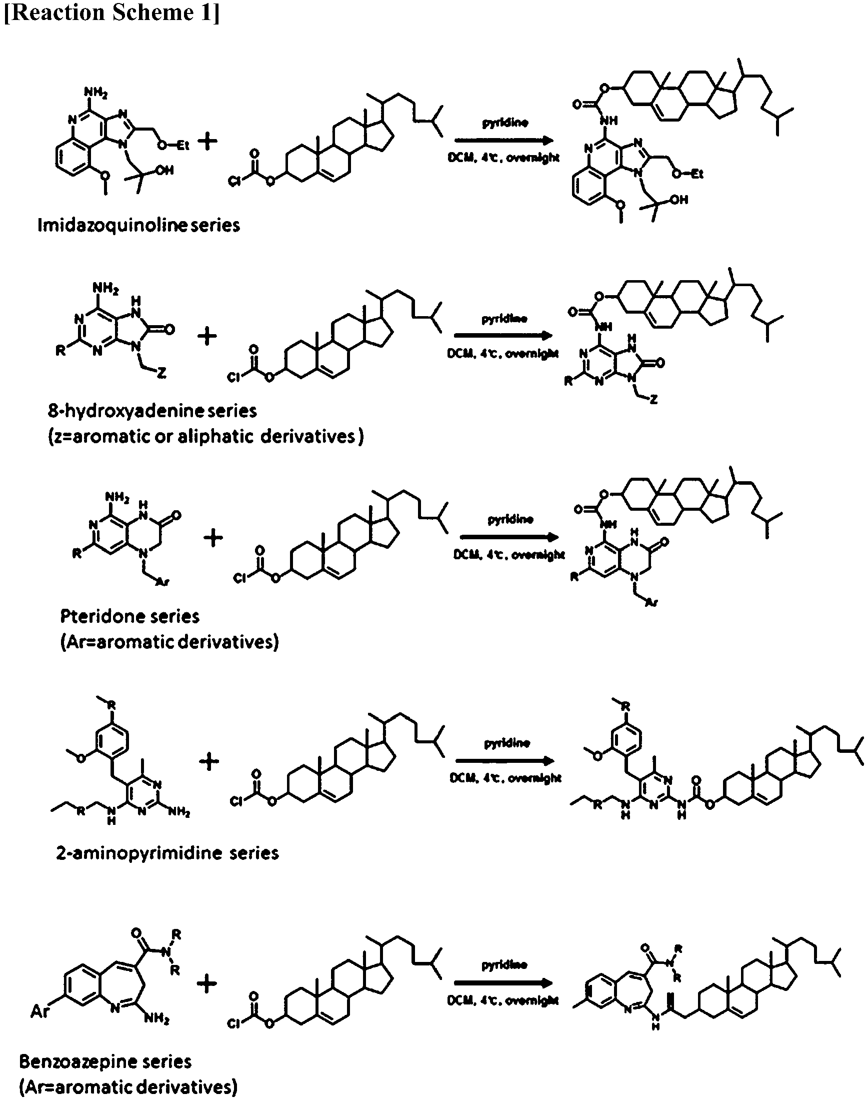

The R is a side chain including an aliphatic or aromatic group, and may include -NH-, - CO-, -CONH-, -CSNH-, -COO-, -CSO-, -SO₂NH-, -SO₂-, -SO-, -O-, and the like.

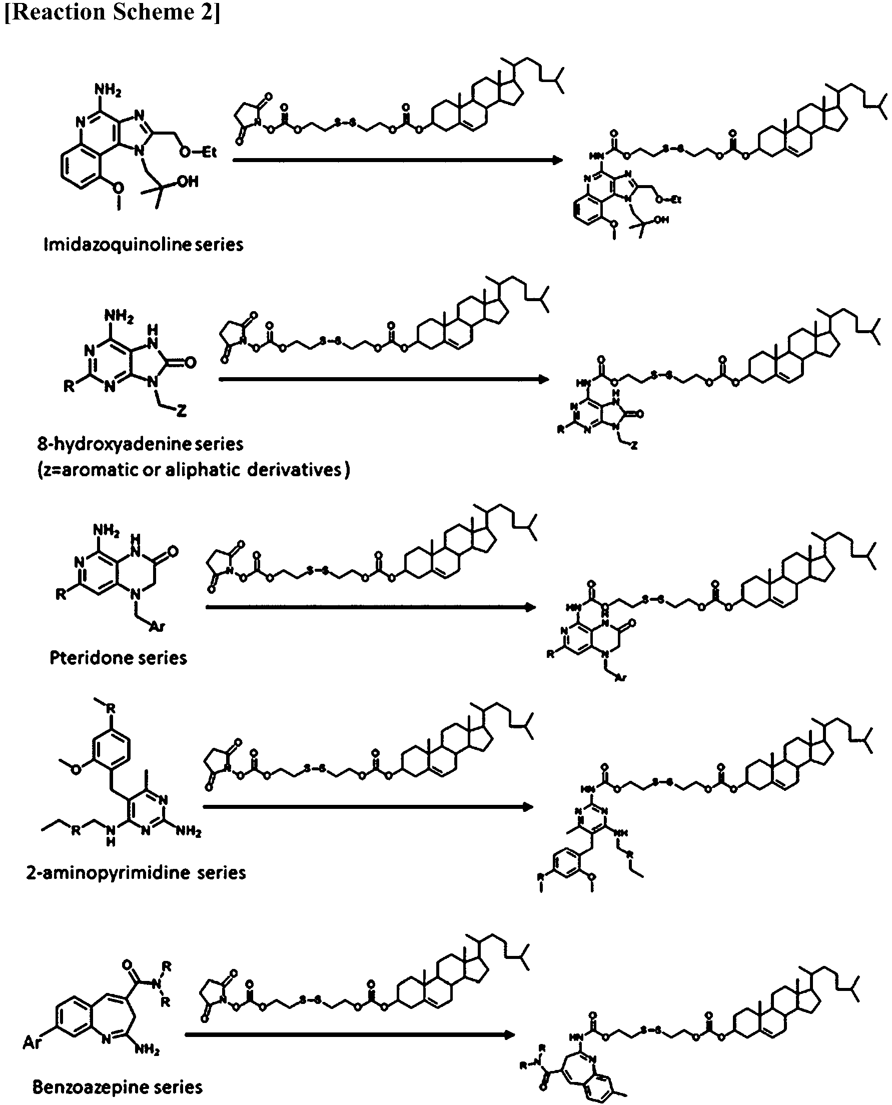

The R is a side chain including an aliphatic or aromatic group, and may include -NH-, - CO-, -CONH-, -CSNH-, -COO-, -CSO-, -SO₂NH-, -SO₂-, -SO-, -O-, and the like.

### 1.1. Synthesis of resiquimod-cholesterol conjugate having carbamate bond

To synthesize a TLR 7/8 agonist conjugated with cholesterol, a conjugate of cholesterol and a TLR 7/8 agonist was synthesized by the method of Reaction Scheme 3 below using resquimod (R848), which is one of the TLR 7/8 agonists. More specifically, 100 µL of pyridine was added to 31.4 mg of resquimod and dissolved in 3 mL of dichloromethane. To this solution, a solution prepared by adding and dissolving 90.0 mg of cholesteryl chloroformate in 1 mL of dichloromethane was slowly added dropwise. Thereafter, the mixed solution was stirred at 4 °C for 16 hours to prepare a mixture. After adjusting the temperature of the resulting mixture to room temperature, distilled water was added to separate the water and dichloromethane layers. Sodium sulfate was then added to the separated dichloromethane layer and allowed to react for 16 hours to remove remaining water. The remaining solution was purified using a silica gel column to obtain cholesterol-conjugated resquimod as white powder. The structures of the resquimod used in the synthesis and the obtained cholesterol-conjugated resquimod were verified using ¹H-NMR and ¹⁵N-heteronuclear single quantum coherence spectroscopy (HSQC).

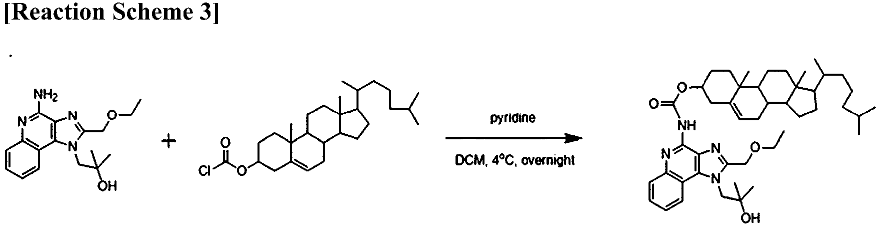

### 1.2. Synthesis of imiquimod-cholesterol conjugate having carbamate bond

To synthesize a TLR 7/8 agonist conjugated with cholesterol, a conjugate of cholesterol and a TLR 7/8 agonist was synthesized by the method of Reaction Scheme 4 below using imiquimod (R837), which is one of the TLR 7/8 agonists. More specifically, 100 µL of pyridine was added to 31.4 mg of imiquimod and dissolved in 3 mL of dichloromethane. To this solution, a solution prepared by adding and dissolving 90.0 mg of cholesteryl chloroformate in 1 mL of dichloromethane was slowly added dropwise. Thereafter, the mixed solution was stirred at 4 °C for 16 hours to prepare a mixture. After adjusting the temperature of the resulting mixture to room temperature, distilled water was added to separate the water and dichloromethane layers. Sodium sulfate was then added to the separated dichloromethane layer and allowed to react for 16 hours to remove remaining water. The remaining solution was purified using a silica gel column to obtain cholesterol-conjugated imiquimod as white powder. The structures of the imiquimod used in the synthesis and the obtained cholesterol-conjugated imiquimod were verified using ¹H-NMR and ¹⁵N-HSQC.

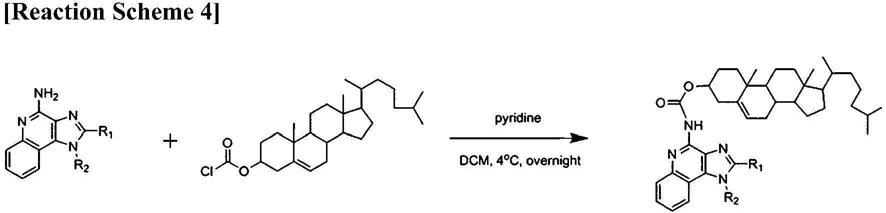

The R₁ or R₂ is a side chain including an aliphatic or aromatic group, and may include - NH-, -CO-, -CONH-, -CSNH-, -COO-, -CSO-, -SO₂NH-, -SO₂-, -SO-, -O-, and the like.

### 1.3. Synthesis of resquimod-cholesterol conjugate crosslinked with disulfide

To synthesize a TLR 7/8 agonist crosslinked with cholesterol-disulfide, the method of Reaction Scheme 5 below was used. More specifically, 1.54 g of 2-hydroxyethyl disulfide was dissolved in 30 mL of tetrahydrofuran and then slowly added dropwise to a phosgene solution (15 mL, 15% by weight in toluene) to prepare a mixture. After the resulting mixture was stirred at 25 °C for 10 hours, the solvent was evaporated under vacuum. Thereafter, 2.3 g of N-hydroxysuccinimide was dissolved in tetrahydrofuran, and the resulting solution was mixed with the above-mentioned mixture, and 1.57 mL of triethylamine was added thereto. After allowing a reaction to occur at 40 °C for 16 hours, precipitates were removed, and the solvent was evaporated under vacuum. After purification using silica gel column chromatography, the product was recrystallized with cold hexane and dried under vacuum to obtain a purified disulfide crosslinking linker as a white solid. In addition, 387 mg of cholesterol was dissolved in 10 mL of dichloromethane, and then 523 mg of the disulfide crosslinking linker was added thereto, followed by stirring at room temperature for 16 hours. Cholesterol-disulfide, in which cholesterol and disulfide are bonded, was purified as white powder using a silica gel column. Thereafter, 31.4 mg of resquimod and 80 mg of the cholesterol-disulfide were added to 5 mL of dichloromethane and stirred at room temperature for 16 hours. Distilled water was added to the stirred solution to separate the water and dichloromethane layers. Sodium sulfate was added to the separated dichloromethane layer and allowed to react for 16 hours to remove remaining water. The remaining solution was purified using a silica gel column to obtain resquimod crosslinked with cholesterol-disulfide as white powder. The structures of the resquimod used in the synthesis and the obtained cholesterol-conjugated resquimod were verified using ¹H-NMR and ¹⁵N-HSQC.

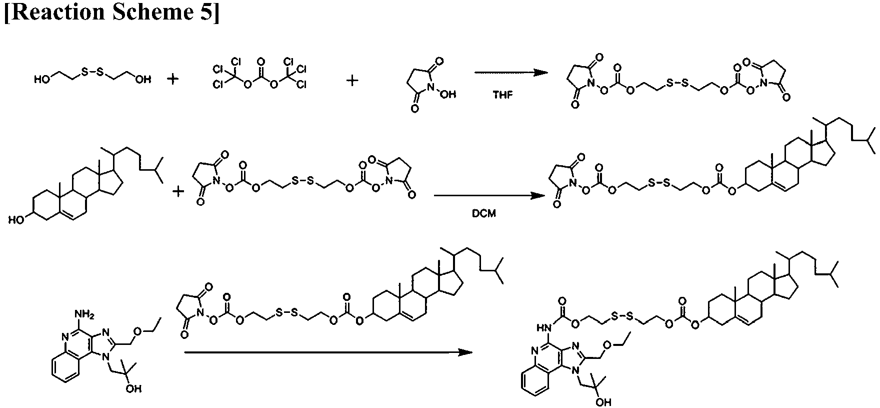

### 1.4. Synthesis of imiquimod-cholesterol conjugate crosslinked with disulfide

To synthesize a TLR 7/8 agonist crosslinked with cholesterol-disulfide, the method of Reaction Scheme 6 below was used. More specifically, 1.54 g of 2-hydroxyethyl disulfide was dissolved in 30 mL of tetrahydrofuran and then slowly added dropwise to a phosgene solution (15 mL, 15% by weight in toluene) to prepare a mixture. After the resulting mixture was stirred at 25 °C for 10 hours, the solvent was evaporated under vacuum. Thereafter, 2.3 g of N-hydroxysuccinimide was dissolved in tetrahydrofuran, and the resulting solution was mixed with the above-mentioned mixture, and 1.57 mL of triethylamine was added thereto. After allowing a reaction to occur at 40 °C for 16 hours, precipitates were removed, and the solvent was evaporated under vacuum. After purification using silica gel column chromatography, the product was recrystallized with cold hexane and dried under vacuum to obtain a purified disulfide crosslinking linker as a white solid. In addition, 387 mg of cholesterol was dissolved in 10 mL of dichloromethane, and then 523 mg of the disulfide crosslinking linker was added thereto, followed by stirring at room temperature for 16 hours. Cholesterol-disulfide, in which cholesterol and disulfide are bonded, was purified as white powder using a silica gel column. Thereafter, 31.4 mg of imiquimod and 80 mg of the cholesterol-disulfide were added to 5 mL of dichloromethane and stirred at room temperature for 16 hours. Distilled water was added to the stirred solution to separate the water and dichloromethane layers. Sodium sulfate was added to the separated dichloromethane layer and allowed to react for 16 hours to remove remaining water. The remaining solution was purified using a silica gel column to obtain imiquimod crosslinked with cholesterol-disulfide as white powder. The structures of the imiquimod used in the synthesis and the obtained cholesterol-conjugated imiquimod were verified using ¹H-NMR and ¹⁵N-HSQC.

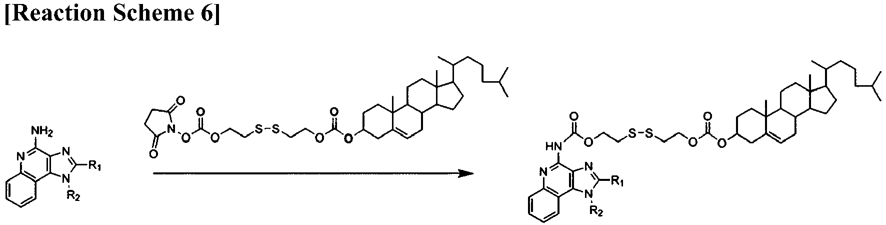

The R₁ or R₂ is a side chain including an aliphatic or aromatic group, and may include - NH-, -CO-, -CONH-, -CSNH-, -COO-, -CSO-, -SO₂NH-, -SO₂-, -SO-, -O-, and the like.

### Example 2: Preparation of nanoparticles including cholesterol-TLR 7/8 agonist conjugate

Cholesterol-TLR 7/8 agonist conjugates were prepared in the form of nanoliposomes to maximize interaction with immune cells. Although nanoliposomes were prepared as one example, cholesterol-TLR 7/8 agonist conjugates comprise cholesterol and thus can be easily loaded, inserted, or encapsulated into various drug delivery systems such as lipid-containing nanoliposomes, nanoemulsions, and nanomicelles, as well as hydrogels and polymer nanoparticles, allowing the conjugates to be manufactured in various drug forms. More specifically, 1,2-dioleoyl-sn-glycero-3-phosphocholine (DOPC) and 1,2-dioleoyl-3-trimethylammonium-propane (DOTAP) were first dissolved using an ED solution (containing 10% by volume of as dimethyl sulfoxide (DMSO) and 90% by volume of ethanol) to reach a concentration of 50 mg/mL each. Thereafter, the resquimod-cholesterol conjugate bonded with a disulfide bond, synthesized in the same manner as in Example 1.3, was dissolved in the ED solution at a concentration of 10 mg/mL to prepare a solution of TLR 7/8 agonist having a temporarily inactivated active site. A lipid stock was prepared by mixing 160 µL of the prepared DOPC solution, 160 µL of the DOTAP solution, 400 µL of the resquimod-cholesterol conjugate solution, and 280 µL of the ED solution. In addition, the lipid stock and a phosphate buffer at pH 6.0 were mounted on an Ignite device from Precision Nanosystems at a ratio of 1:3. Nanoliposomes were prepared under conditions of a syringe volume of 3 mL, a flow rate ratio of 3:1, a total volume of 3.5 mL, and a total flow rate of 12 mL/min. The prepared liposomes were filtered using a 0.2 µm syringe filter and then dialyzed using 10K Dialysis Cassettes at 160 rpm for two to four hours. The buffer was replaced, and dialysis was performed for another 16 hours to remove the organic solvents used during the preparation. The prepared nanoliposomes (ProLNG-001) were stored under refrigeration and protected from light until use.

### Example 3: Confirmation of effect of combination therapy of TLR 7/8 agonist having temporarily inactivated active site and anti-cancer drug

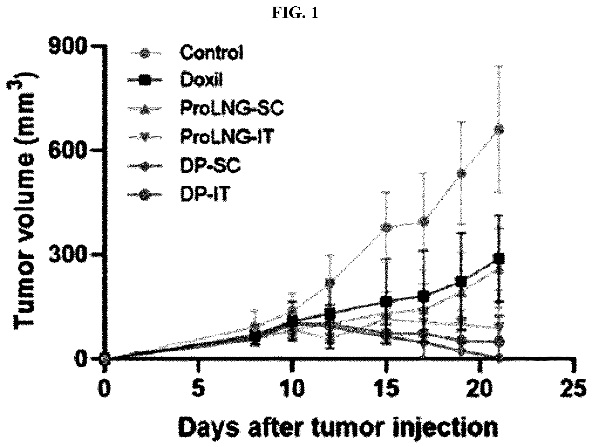

To confirm an effective combination therapy of a temporarily inactivated cholesterol-TLR 7/8 agonist and an anti-cancer drug, ProLNG-001 prepared in the same manner as in Example 2 and an anti-cancer drug were co-administered under various conditions. Hereinafter, all animal studies were performed in accordance with the Institutional Animal Care and Use Committee (IACUC) guidelines. First, a syngeneic mouse tumor model was prepared by subcutaneously injecting 5X10⁵ 4T1 cells, which are a triple-negative breast cancer (TNBC) cell line, into the back of seven to eight-week-old BALB/c mice. Animal housing and experiments were all conducted in a specific pathogen free SPF facility, and each experimental group consisted of eight mice. Experimental groups for confirming the effect of co-administration were intravenously (IV) injected with 80 µg of Doxil^{ⓡ}, which is widely used as an anti-cancer drug, on day 7 after the transplantation of the 4T1 cell line. On day 8, 140 µg of ProLNG-001 was administered via intratumoral (IT) or subcutaneous (SC) injection. On day 11, 140 µg of ProLNG-001 was again administered via intratumoral or subcutaneous injection to complete the first round of administration. In addition, for the second round of administration, 80 µg of Doxil^{ⓡ} was intravenously injected on day 14, followed by 140 µg of ProLNG-001 via intratumoral or subcutaneous injection on day 15, and another 140 µg of ProLNG-001 via intratumoral or subcutaneous injection on day 18. Experimental groups for confirming the effect of monotherapy were transplanted with the 4T1 cell line and then injected with Doxil^{ⓡ} or ProLNG-001 following the same schedule as the co-administration group. The Doxil^{ⓡ} monotherapy group was injected with an equal volume of phosphate buffered saline at the time of ProLNG-001 injection, and the ProLNG-001 monotherapy group was injected with an equal volume of phosphate buffered saline at the time of Doxil^{ⓡ} injection. The control group was injected only with an equal volume of phosphate buffered saline at each administration time point. In addition, tumor size was measured every two to three days until day 21. The tumor size was calculated by measuring the horizontal and vertical lengths of the tumor using a caliper and substituting the measured lengths into the formula below. All subsequent results were expressed as mean value ± standard deviation. Statistical significance was confirmed by Student's t-test, and a p-value less than 0.05 was considered statistically significant. The results are shown in FIG. 1. $V = \frac{{Width}^{2} \times Length}{2}$

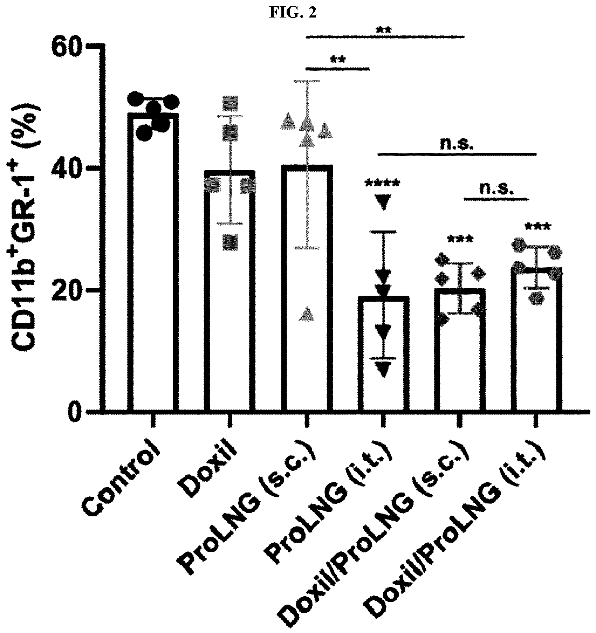

In addition, after completing the two rounds of administration, the mice were euthanized on day 21, and lymph nodes, tumor tissues, and spleens were isolated. The isolated tumor tissues were initially minced using scissors, treated with 1 mg/mL of collagenase type I, and allowed to react at 37 °C for one hour to dissociate the tissues into single cells. The dissociated cells were filtered using a 70 µm strainer and washed with phosphate buffered saline. The obtained spleens or lymph nodes were initially minced using scissors and treated with red blood cell lysis buffer at 37 °C for 10 minutes to lyse red blood cells. The resulting mixture was then filtered using a 70 µm strainer and washed with phosphate buffered saline. The washed tumor cells, spleen cells, and lymph node cells were each labeled using antibodies. Myeloid-derived suppressor cells (MDSCs) in the spleen were labeled using anti-CD11b and anti-GR-1 antibodies. T cells in the spleen were labeled using anti-CD3 antibodies, or anti-CD4 and anti-TNF-α antibodies, or anti-CD8 and anti-INF-γ antibodies. T cells in the tumor tissue were labeled using anti-CD3 or anti-CD49b antibodies. Natural killer (NK) cells were labeled by marking CD3-positive T cells with anti-CD69 antibodies, or CD49b-positive cells with anti-CD69 antibodies. In addition, labeling was performed using anti-PD-L1 antibodies. The samples were then analyzed using a flow cytometer. The results are shown in FIGS. 2 to 4.

As shown in FIG. 1, the Doxil^{ⓡ} monotherapy group (Doxil) and the ProLNG-001 subcutaneous injection group (ProLNG-SC) showed inhibited tumor growth compared to the control group, but the tumors continued to grow gradually. However, it was confirmed that the tumor size consistently decreased in the ProLNG-001 intratumoral injection group (ProLNG-IT), the Doxil^{ⓡ} and ProLNG-001 subcutaneous injection co-administration group (DP-SC), and the Doxil^{ⓡ} and ProLNG-001 intratumoral injection co-administration group (DP-IT). Particularly, it was confirmed that almost no tumor growth occurred in the case of DP-IT.

In addition, as shown in FIG. 2, the number of MDSC cells, which perform immunosuppressive functions in the spleen, was not significantly reduced in the control, Doxil, and ProLNG-SC groups. However, a significant decrease was confirmed in the ProLNG-001 intratumoral injection group (ProLNG (i.t.)), the Doxil^{ⓡ} and ProLNG-001 subcutaneous injection co-administration group (Doxil/ProLNG (s.c.)), and the Doxil^{ⓡ} and ProLNG-001 intratumoral injection co-administration group (Doxil/ProLNG (i.t.)).

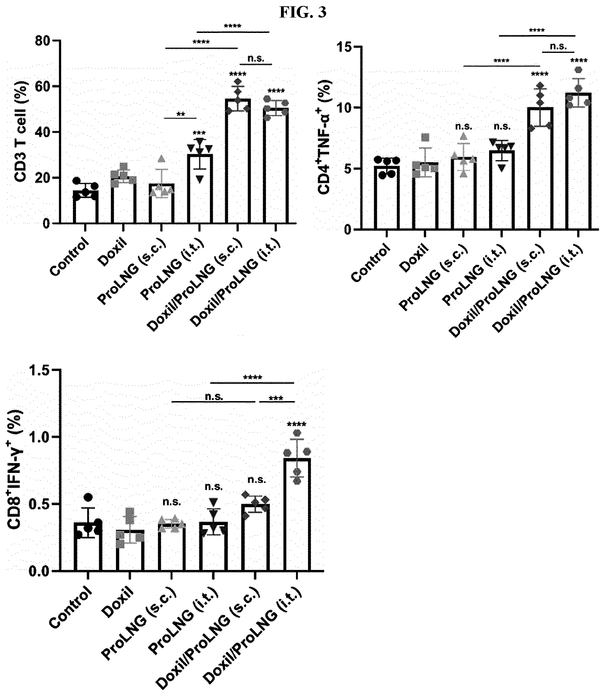

As shown in FIG. 3, the numbers of CD3+ T cells, CD4+TNF-α+ T cells, and CD8+INF-γ+ T cells in the spleen also showed an increasing trend in the ProLNG (i.t.), Doxil/ProLNG (s.c.), Doxil/ProLNG (i.t.) groups. In the case of the Doxil/ProLNG (i.t.) group, a remarkably high number of T cells were confirmed.

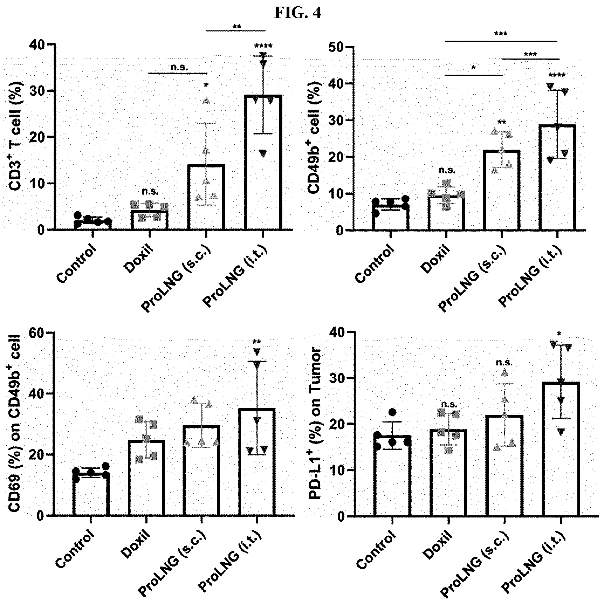

Furthermore, as shown in FIG. 4, it was confirmed that the numbers of CD3+ T cells and NK cells, which exhibit anti-cancer effects within tumor tissue, were significantly increased in the ProLNG (i.t.) group.

The above-described results confirmed that intratumoral administration of a TLR 7/8 agonist with a temporarily inactivated active site can effectively increase the immune activation effect and that the synergistic effect of co-administration can be increased by administering an inactivated TLR 7/8 agonist that exhibits immune activation effects after the administration of an anti-cancer drug.

### Example 4: Confirmation of effect of combination therapy of TLR 7/8 agonist having temporarily inactivated active site, anti-cancer drug, and immune checkpoint inhibitor

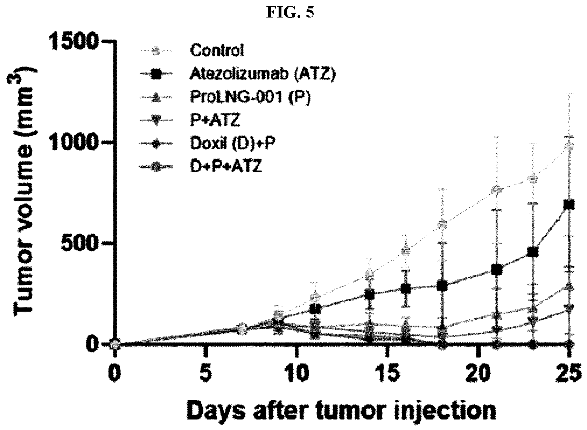

To confirm the effect of combination therapy with an immune checkpoint inhibitor, an experiment was conducted in the same manner as in Example 3. Briefly, on day 7 after the transplantation of the 4T1 cell line, 80 µg of Doxil^{ⓡ} was intravenously injected, and 200 µg of anti-PD-L1 (atezolizumab; ATZ), which is widely used as an immune checkpoint inhibitor, was intraperitoneally injected (IP). On day 8, 140 µg of ProLNG-001 was administered via intratumoral injection. On day 9, 200 µg of ATZ was intraperitoneally injected. On day 11, 140 µg of ProLNG-001 was again administered via intratumoral injection, and 200 µg of ATZ was intraperitoneally injected to complete the first round of administration. For the second round of administration, 80 µg of Doxil^{ⓡ} was intravenously injected and 200 µg of ATZ was intraperitoneally injected on day 14. On day 15, 140 µg of ProLNG-001 was administered via intratumoral injection. On day 16, 200 µg of ATZ was intraperitoneally injected. On day 18, 140 µg of ProLNG-001 was administered via intratumoral injection and 200 µg of ATZ was intraperitoneally injected. All other conditions were the same as in Example 3. Tumor size was measured every two to three days until day 25. The results are shown in FIG. 5.

As shown in FIG. 5, while the tumor size in the ATZ monotherapy group increased similarly to the control group, inhibition of tumor growth was confirmed in the ProLNG-001 monotherapy group (ProLNG-001 P) and the ProLNG-001 and ATZ co-administration group (P+ATZ). In addition, it was confirmed that almost no tumor growth occurred in the Doxil^{ⓡ} and ProLNG-001 co-administration group (D+P) and the Doxil^{ⓡ}, ProLNG-001, and ATZ triple co-administration group (D+P+ATZ). These results confirmed that the anti-cancer effect can be significantly increased by combining an immune checkpoint inhibitor with the co-administration of an anti-cancer drug and a TLR 7/8 agonist having a temporarily inactivated active site.

### Example 5: Confirmation of anti-cancer effect in various cancer types

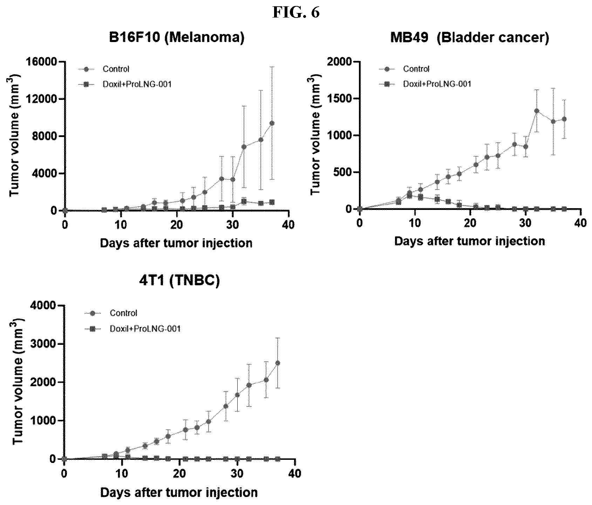

To confirm whether the same anti-cancer effect is exhibited in various cancer types, the anti-cancer effect was verified in the same manner as in Example 3 using the bladder cancer cell line MB49 and the melanoma cell line B16F10. More specifically, 5X10⁵ MB49 and 5X10⁵ B16F10 cells were each subcutaneously injected into the back of seven to eight-week-old C58BL/6 mice, and 5X10⁵ 4T1 cells were subcutaneously injected into the back of seven to eight-week-old BALB/c mice to prepare mouse tumor models. The control group was injected with an equal volume of phosphate buffered saline, and the co-administration group was administered 80 µg of Doxil^{ⓡ} via intravenous injection and 140 µg of ProLNG-001 via intratumoral injection according to the same schedule as in Example 3. All three types of tumors were confirmed to have reached a size of 100 mm³ on day 7 after transplantation, and then the experiment was conducted. The results are shown in FIG. 6.

As shown in FIG. 6, it was confirmed that tumor growth was almost completely inhibited in the melanoma model, and in the breast cancer and bladder cancer models, the tumors did not grow at all even over a long period of about 40 days. These results confirmed that the co-administration of the present invention can effectively inhibit tumor growth in various cancer types.

### Example 6: Confirmation of anti-cancer effect in recurrent cancer

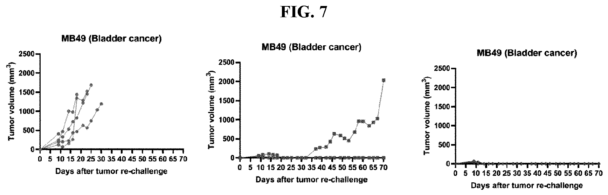

To confirm whether the anti-cancer effect is exhibited in recurrent cancer, an experiment was conducted using the bladder cancer cell line MB49 in the same manner as in Example 5. For the experimental groups that achieved complete response (CR), a rechallenge was performed approximately 50 days after the initial tumor cell transplantation by subcutaneously injecting 5X10⁵ cells into the back of six mice. In three of these mice, 80 µg of Doxil^{ⓡ} was administered via intravenous injection and 140 µg of ProLNG-001 was administered via intratumoral injection for co-administration according to the same schedule as in Example 3. The remaining 3 mice were injected with an equal volume of phosphate buffered saline. The results are shown in FIG. 7.

In FIG. 7, the left graph shows the results of a control group of four mice where the MB49 bladder cancer cell line was transplanted into new mice and phosphate buffered saline was injected. The middle graph shows the results of three mice from the group that previously achieved CR, which were re-transplanted with the MB49 cell line and injected with phosphate buffered saline. The right graph shows the results of three mice from the group that previously achieved CR, which were re-transplanted with the MB49 cell line and received co-administration according to the same schedule as in Example 3. Since all the mice in the initial control group had died, the experiment for the control was conducted using new mice without re-transplantation. As shown in the right graph of FIG. 7, in the experimental group that was re-transplanted with the tumor cell line and received co-administration, the tumor seemed to grow slightly at first, but the growth was inhibited again, and no growth was observed up to 70 days after re-transplantation. Furthermore, in two out of the three individuals that were injected with an equal volume of phosphate buffered saline without receiving treatment after re-transplantation, the cancer began to grow but then the growth was inhibited, confirming that CR occurred up to day 70.

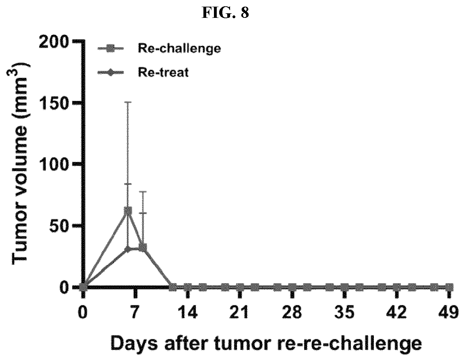

On day 72 after the rechallenge, five mice in which CR was confirmed were subjected to a second rechallenge by subcutaneously injecting 5X10⁵ cells of the MB49 cell line into the back of the mice. Thereafter, the growth of the cancer was observed without any further treatment. The results are shown in FIG. 8.

As shown in FIG. 8, in both the three mice that received anti-cancer treatment after the first rechallenge (Re-treat) and the two mice that did not receive treatment (Re-challenge), the cancer appeared to grow for about a week, but the cancer cells did not grow at all starting from approximately week 2. These results confirm that the co-administration of the present invention is not merely a simple anti-cancer treatment. Instead, tumor lysates generated through the primary anti-cancer treatment that kills cancer cells act as antigens, and the subsequently injected inactivated TLR 7/8 agonist acts as an adjuvant. This effectively increases the immune response in the body so that the co-administration can not only achieve the primary anti-cancer therapy, but also effectively inhibit cancer recurrence even without additional treatment.

### Example 7: Confirmation of anti-cancer effect according to administered dose

To confirm the anti-cancer effect according to the administered dose, an experiment was conducted in the same manner as in Example 3. However, to confirm the anti-cancer effect based on the dose of the temporarily inactivated TLR 7/8 agonist, ProLNG-001 was administered via intratumoral injection at doses of 7, 14, 28, 35, 70, or 140 µg. The results are shown in FIG. 9.

As shown in FIG. 9, it was confirmed that the anti-cancer effect increased as the administered dose of ProLNG-001 increased, reaching a plateau at concentrations of 35 µg or higher. These results confirm that a specific dose or higher is required to exhibit a significant anti-cancer effect in treatment using a temporarily inactivated TLR 7/8 agonist.

### Example 8: Confirmation of anti-cancer effect according to administration site

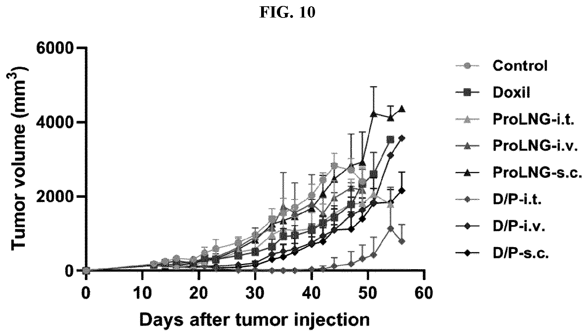

To confirm the anti-cancer effect according to the administration site of the temporarily inactivated TLR 7/8 agonist, a syngeneic mouse tumor model was prepared by subcutaneously injecting 5X10⁵ 4T1 cells into the back of seven to eight-week-old BALB/c mice. While anti-cancer treatment was initiated when the tumor size reached 100 mm³, that is, on day 7, in the previous examples, in this example, treatment was started on day 12 when the tumor size reached 150 mm³ to mimic a more advanced cancer stage. More specifically, 80 µg of Doxil^{ⓡ} was administered via intravenous injection on day 12. On day 13, 140 µg of ProLNG-001 was administered via intratumoral (i.t.), intravenous (i.v.), or subcutaneous injection (s.c.). On day 16, 140 µg of ProLNG-001 was injected again by the same method to complete the first round of administration. For the second round of administration, 80 µg of Doxil^{ⓡ} was administered via intravenous injection on day 19, and 140 µg of ProLNG-001 was injected on days 20 and 23 using the same method as before. For the group to confirm the effect of monotherapy, Doxil^{ⓡ} or ProLNG-001 was injected according to the same schedule as the co-administration group. The Doxil^{ⓡ} monotherapy group received an equal volume of phosphate buffered saline at the time of ProLNG-001 injection, and the ProLNG-001 monotherapy group received an equal volume of phosphate buffered saline at the time of Doxil^{ⓡ} injection. The control group was injected only with an equal volume of phosphate buffered saline at each time point. The results are shown in FIG. 10.

As shown in FIG. 10, it was confirmed that, compared to the monotherapy groups, tumor growth was inhibited in the co-administration groups. In particular, it was confirmed that when the temporarily inactivated TLR 7/8 agonist was administered via intratumoral injection, tumor growth was inhibited even until after day 50. These results confirm that the co-administration method of the present invention can be used to treat cancer not only in early stages but also in advanced stages, and that the anti-cancer effect is most effectively increased when the temporarily inactivated TLR 7/8 agonist is administered intratumorally.

### Example 9: Confirmation of anti-cancer effect according to administered dose and administration schedule

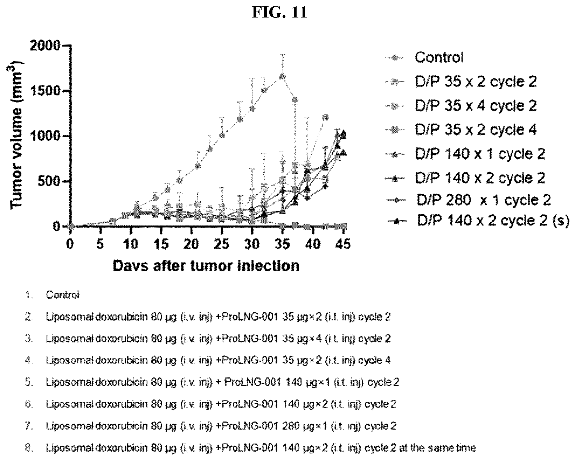

To confirm the anti-cancer effect according to the dose and administration schedule of the temporarily inactivated TLR 7/8 agonist, 4T1 cells were subcutaneously injected into the back of mice in the same manner as in Example 3, and anti-cancer treatment was initiated when the tumor size reached 100 mm³. ProLNG-001 was administered via intratumoral injection at doses of 35, 140, or 280 µg, and the treatment schedules were conducted as shown in FIG. 11. The results of the anti-cancer treatment are shown in FIG. 11.

In FIG. 11, Experimental Group 1 is a group administered phosphate buffered saline; Experimental Group 2 is a group administered an anti-cancer drug and then administered 35 µg of ProLNG-001 two times, on days 1 and 4, with the schedule repeated for two cycles (D/P 35 x 2 cycle 2); Experimental Group 3 is a group administered an anti-cancer drug and then administered 35 µg of ProLNG-001 four times, on days 1, 2, 3, and 4, with the schedule repeated for two cycles (D/P 35 x 4 cycle 2); Experimental Group 4 is a group administered an anti-cancer drug and then administered 35 µg of ProLNG-001 two times, on days 1 and 4, with the schedule repeated for four cycles (D/P 35 x 2 cycle 4); Experimental Group 5 is a group administered an anti-cancer drug and then administered 140 µg of ProLNG-001 once, on day 1, with the schedule repeated for two cycles (D/P 140 x 1 cycle 2); Experimental Group 6 is a group administered an anti-cancer drug and then administered 140 µg of ProLNG-001 two times, on days 1 and 4, with the schedule repeated for two cycles (D/P 140 x 2 cycle 2); Experimental Group 7 is a group administered an anti-cancer drug and then administered 280 µg of ProLNG-001 once, on day 1, with the schedule repeated for two cycles (D/P 280 x 1 cycle 2); and Experimental Group 8 is a group administered an anti-cancer drug and 140 µg of ProLNG-001 simultaneously, and then administered another 140 µg of ProLNG-001 on day 4, with the schedule repeated for two cycles (D/P 140 x 2 cycle 2(s)). The anti-cancer drug was administered repeatedly at one-week intervals. As shown in FIG. 11, it was confirmed that long-term repeated treatment with lower doses can significantly increase the anti-cancer effect compared to short-term treatment with higher doses. These results confirmed that repeated administration exhibits an effect similar to that of a booster shot of a vaccine, significantly improving the cancer treatment effect by boosting the immune response.

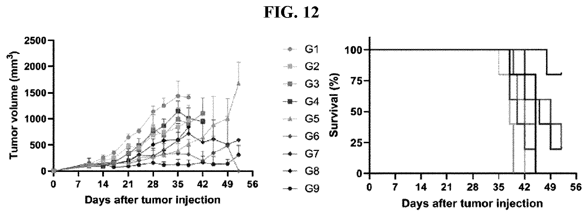

To further clarify the effect according to the administration schedule, experiments were conducted using various administration schedules. Anti-cancer treatment was initiated when the tumor size reached 100 mm³. More specifically, the tumor size and survival rate were confirmed using the following groups: G1 represents a group administered an equal volume of phosphate buffered saline on each administration day; G2 represents a group administered 80 µg of Doxil^{ⓡ} i.v. seven times at one-week intervals; G3 represents a group administered 80 µg of Doxil^{ⓡ} i.v. three times at two-week intervals; G4 represents a group administered 80 µg of Doxil^{ⓡ} i.v. twice at three-week intervals; G5 represents a group administered 80 µg of Doxil^{ⓡ} i.v. and then administered 35 µg of ProLNG-001 i.t. the next day, with the schedule repeated for seven cycles at one-week intervals; G6 represents a group administered 80 µg of Doxil^{ⓡ} i.v. and then administered 140 µg of ProLNG-001 i.t. the next day, with the schedule repeated for seven cycles at one-week intervals; G7 represent a group administered 80 µg of Doxil^{ⓡ} i.v. and then administered 140 µg of ProLNG-001 i.t. the next day, with the schedule repeated for four cycles at two-week intervals; G8 represents a group administered 80 µg of Doxil^{ⓡ} i.v. and then administered 140 µg of ProLNG-001 i.t. the next day, with the schedule repeated for three cycles at three-week intervals; G9 represents a group administered 80 µg of Doxil^{ⓡ} i.v., administered 140 µg of ProLNG-001 i.t. the next day, and then administered another 140 µg of ProLNG-001 three days later, with the schedule repeated for seven cycles at one-week intervals. The results are shown in FIG. 12.

As shown in FIG. 12, it was also confirmed the anti-cancer effect increased in the experimental groups administered at one-week intervals and that co-administration with a temporarily inactivated TLR 7/8 agonist can increase the therapeutic effect compared to using the anti-cancer drug doxorubicin alone. Furthermore, it was also confirmed that the experimental group administered the anti-cancer drug followed by the administration of the temporarily inactivated TLR 7/8 agonist two times exhibited the most significant therapeutic effect.

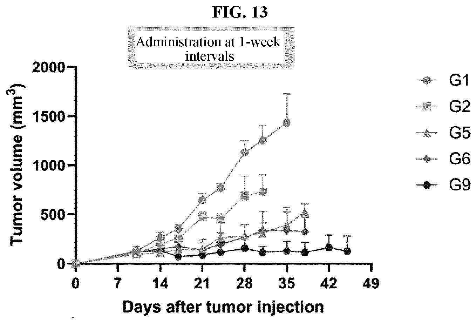

To demonstrate this more clearly, FIG. 13 shows the tumor sizes of the experimental groups that underwent the anti-cancer treatment at one-week intervals, recorded until the first death occurred.

As shown in FIG. 13, it was confirmed that the anti-cancer therapeutic effect can be enhanced when co-administration of the anti-cancer drug and the temporarily inactivated TLR 7/8 agonist is performed at one-week intervals and that the group administered the temporarily inactivated TLR 7/8 agonist two times exhibited the most significant therapeutic effect.

### Example 10: Confirmation of immune response by anti-cancer treatment

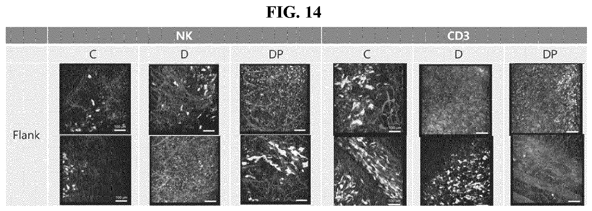

To confirm the intratumoral immune response according to the administration of the temporarily inactivated TLR 7/8 agonist, 4T1 cells were subcutaneously injected into the flank of mice in the same manner as in Example 3. Anti-cancer treatment was initiated when the tumor size reached 100 mm³. The anti-cancer treatment was conducted for: a group (D) administered 80 µg of Doxil^{ⓡ} (D); a group (DP) administered 80 µg of Doxil^{ⓡ}, administered 140 µg of ProLNG-001 via i.t. the next day, and then administered another 140 µg of ProLNG-001 i.t. three days later; and a control group (C) administered phosphate buffered saline. Observations were made using a fluorescence microscope after the second administration of ProLNG-001. Fluorescence observation was commissioned to IVIM Technology Inc. 4T1 cells were observed at a green fluorescence wavelength, CD3-positive T cells and CD49-positive cells, which are NK cells, were observed at a red fluorescence wavelength, and CD31-positive vascular endothelial cells were observed at a blue fluorescence wavelength. The results are shown in FIG. 14.

As shown in FIG. 14, in the control group, there was almost no change in the morphology of cancer cells, and many CD49b-positive cells were observed around the blood vessels. Similarly, the Doxil^{ⓡ} group showed almost no change in cancer cell morphology, with excessive neovascularization around the cells and CD49b-positive cells observed near the vessels. On the other hand, in the DP group, the cells were observed not as normal cells but as dead cancer cells, and co-localization of CD49b-positive cells within the blood vessels or with the cancer cells was confirmed. These results confirmed that the co-administration of the present invention activates the immune response in the body, inducing the infiltration of immune cells such as T cells and NK cells into the tumor.

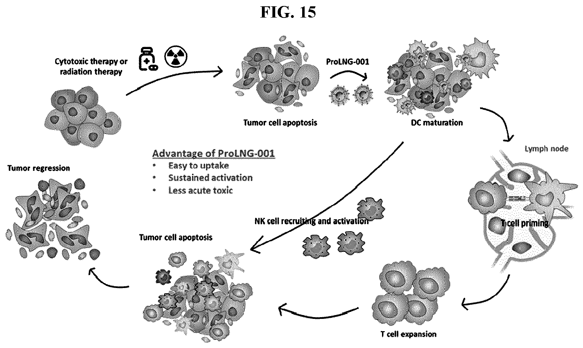

The above-described results, as shown in FIG. 15, confirmed that the co-administration of the anti-cancer drug and the TLR 7/8 agonist having a temporarily inactivated active site significantly enhances the therapeutic effect by initially inducing cancer cell death and destruction of tumor tissue through primary treatments such as chemical chemotherapy, radiotherapy, or oncolytic virus administration, then enabling the resulting tumor lysates to act as antigens, enhancing the immunological therapeutic effect of the cancer treatment. At this time, the administration of the TLR 7/8 agonist having a temporarily inactivated active site allows the agonist to act as an adjuvant. Through the immune-activating action, the agonist induces a more potent immuno-oncological effect, thereby significantly increasing the overall anti-cancer efficacy. In other words, the above-described results confirmed that the therapeutic effect can be significantly enhanced by utilizing the time interval between the primary anti-cancer treatment and the secondary immuno-oncological activation of the TLR 7/8 agonist. Furthermore, it was also confirmed that the anti-cancer effect can be even more significantly improved by intratumoral injection of the temporarily inactivated TLR 7/8 agonist or by additional administration of an immune checkpoint inhibitor. Therefore, by utilizing a TLR 7/8 agonist that reduces side effects through temporary inactivation while maintaining immune activation for a longer period, the present invention provides a method of significantly enhancing anti-cancer therapeutic effects. Therefore, the present invention can be effectively utilized for the treatment of various types of cancer.

The foregoing description of the present invention is intended for illustrative purposes, and it will be understood by those skilled in the art that various modifications can be made thereto in other specific forms without departing from the technical spirit or essential features of the present invention. Therefore, the embodiments described above should be understood as illustrative in all respects and not restrictive.

### [Industrial Applicability]

The pharmaceutical composition and/or kit according to the present invention can effectively enhance the immune response within a tumor, and thus can not only significantly improve the therapeutic effects against various types of cancer when co-administered with conventional anticancer therapies, but can also effectively suppress recurrence. Accordingly, the pharmaceutical composition and/or kit according to the present invention is expected to be effectively applicable to the treatment of various cancers.

## Claims

1. A pharmaceutical composition for combination administration for preventing or treating cancer, comprising as an active ingredient a temporarily inactivated Toll-like receptor 7/8 agonist in which cholesterol is bonded to an active site via a cleavable linker, wherein the pharmaceutical composition is administered 1 to 20 days after anti-cancer treatment.

2. The pharmaceutical composition of claim 1, wherein the anti-cancer treatment is anti-cancer drug treatment, radiotherapy, or oncolytic virus treatment, which is capable of inducing destruction of tumor tissue.

3. The pharmaceutical composition of claim 2, wherein the anti-cancer drug is one or more selected from the group consisting of doxorubicin, cisplatin, carboplatin, nedaplatin, goserelin, medroxyprogesterone, cyproterone, vinorelbine, cabazitaxel, denosumab, gemcitabine, capecitabine, oxaliplatin, vorinostat, entinostat, 5-fluorouracil, taxol, topotecan, irinotecan, and pharmaceutically acceptable salts thereof.

4. The pharmaceutical composition of claim 2, wherein the oncolytic virus is any one selected from the group consisting of herpesvirus, adenovirus, vaccinia virus, poliovirus, measles virus, vesicular stomatitis virus, reovirus, and genetically modified viruses thereof.

5. The pharmaceutical composition of claim 1, wherein the pharmaceutical composition is administered in the form of an intravenous injection, an intradermal injection, an intratumoral injection, or a subcutaneous injection.

6. The pharmaceutical composition of claim 1, wherein the cancer is any one selected from the group consisting of breast cancer, colorectal cancer, rectal cancer, lung cancer, colon cancer, thyroid cancer, oral cancer, pharyngeal cancer, laryngeal cancer, cervical cancer, brain cancer, ovarian cancer, bladder cancer, kidney cancer, liver cancer, pancreatic cancer, prostate cancer, skin cancer, tongue cancer, uterine cancer, gastric cancer, bone cancer, blood cancer, head and neck cancer, vaginal cancer, esophageal cancer, lymphoma, gallbladder cancer, endocrine gland cancer, adrenal cancer, and melanoma.

7. The pharmaceutical composition of claim 1, wherein the Toll-like receptor 7/8 agonist is one or more selected from the group consisting of an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist.

8. The pharmaceutical composition of claim 1, wherein the cleavable linker comprises one or more bonds selected from the group consisting of a carbamate bond, a disulfide bond, an ester bond, a peptide bond, an azide bond, and combinations thereof.

9. The pharmaceutical composition of claim 1, wherein in the temporary inactivation, a chemical bond with cholesterol is cleaved in response to a tumor microenvironment, enzymes of intracellular endosomes and lysosomes, or pH, and an active site of the Toll-like receptor 7/8 agonist is exposed and a function thereof is restored.

10. The pharmaceutical composition of claim 1, wherein the pharmaceutical composition further comprises an immune checkpoint inhibitor.

11. The pharmaceutical composition of claim 10, wherein the immune checkpoint inhibitor is one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, anti-TIGIT, and the like.

12. The pharmaceutical composition of claim 1, wherein the pharmaceutical composition inhibits the proliferation, metastasis, or recurrence of cancer, or resistance to anti-cancer treatment regimen.

13. A kit for preventing or treating cancer, comprising:
a) a first pharmaceutical composition comprising, as an active ingredient, a substance for anti-cancer treatment; and
b) a second pharmaceutical composition comprising, as an active ingredient, a temporarily inactivated Toll-like receptor 7/8 agonist in which cholesterol is bonded to an active site via a cleavable linker,
wherein the first pharmaceutical composition and the second pharmaceutical composition are sequentially administered.

14. The kit of claim 13, wherein in the sequential administration, the second pharmaceutical composition is administered 1 to 20 days after the first pharmaceutical composition is administered.

15. The kit of claim 13, wherein the second pharmaceutical composition is administered 1 to 10 times.

16. The kit of claim 13, wherein the second pharmaceutical composition is administered in the form of an intravenous injection, an intradermal injection, an intratumoral injection, or a subcutaneous injection.

17. The kit of claim 13, wherein the substance for anti-cancer treatment is an anti-cancer drug, an oncolytic virus, or a radioisotope.

18. The kit of claim 17, wherein the anti-cancer drug is one or more selected from the group consisting of doxorubicin, cisplatin, carboplatin, nedaplatin, goserelin, medroxyprogesterone, cyproterone, vinorelbine, cabazitaxel, denosumab, gemcitabine, capecitabine, oxaliplatin, vorinostat, entinostat, 5-fluorouracil, taxol, topotecan, irinotecan, and pharmaceutically acceptable salts thereof.

19. The kit of claim 17, wherein the oncolytic virus is any one selected from the group consisting of herpesvirus, adenovirus, vaccinia virus, poliovirus, measles virus, vesicular stomatitis virus, reovirus, and genetically modified viruses thereof.

20. The kit of claim 17, wherein the radioisotope is iridium-192.

21. The kit of claim 13, wherein the cancer is any one selected from the group consisting of breast cancer, colorectal cancer, rectal cancer, lung cancer, colon cancer, thyroid cancer, oral cancer, pharyngeal cancer, laryngeal cancer, cervical cancer, brain cancer, ovarian cancer, bladder cancer, kidney cancer, liver cancer, pancreatic cancer, prostate cancer, skin cancer, tongue cancer, uterine cancer, gastric cancer, bone cancer, blood cancer, head and neck cancer, vaginal cancer, esophageal cancer, lymphoma, gallbladder cancer, endocrine gland cancer, adrenal cancer, and melanoma.

22. The kit of claim 13, wherein the Toll-like receptor 7/8 agonist is one or more selected from the group consisting of an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist.

23. The kit of claim 13, wherein the cleavable linker comprises one or more bonds selected from the group consisting of a carbamate bond, a disulfide bond, an ester bond, a peptide bond, an azide bond, and combinations thereof.

24. The kit of claim 13, wherein in the temporary inactivation, a chemical bond with cholesterol is cleaved in response to a tumor microenvironment, enzymes of intracellular endosomes and lysosomes, or pH, and an active site of the Toll-like receptor 7/8 agonist is exposed and a function thereof is restored.

25. The kit of claim 13, wherein the kit further comprises c) a third pharmaceutical composition comprising, as an active ingredient, an immune checkpoint inhibitor.

26. The kit of claim 25, wherein the immune checkpoint inhibitor is one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, anti-TIGIT, and the like.

27. The kit of claim 13, wherein the pharmaceutical composition inhibits the proliferation, metastasis, or recurrence of cancer, or resistance to anti-cancer treatment regimen.

28. A method of preventing or treating cancer, comprising:
a) a step of administering a therapeutically effective amount of a first pharmaceutical composition comprising, as an active ingredient, a substance for anti-cancer treatment to a subject in need thereof; and
b) a step of administering to the subject, 1 to 20 days after the administration of the first pharmaceutical composition, a therapeutically effective amount of a second pharmaceutical composition comprising, as an active ingredient, a temporarily inactivated Toll-like receptor 7/8 agonist in which cholesterol is bonded to an active site via a cleavable linker.

29. A use of a temporarily inactivated Toll-like receptor 7/8 agonist in which cholesterol is bonded to an active site via a cleavable linker, for increasing a therapeutic effect of an anti-cancer treatment by administration thereof 1 to 20 days after the anti-cancer treatment.

30. A use of a temporarily inactivated Toll-like receptor 7/8 agonist in which cholesterol is bonded to an active site via a cleavable linker, for producing a medicament used to increase a therapeutic effect of an anti-cancer treatment by administration thereof 1 to 20 days after the anti-cancer treatment.
